Europäisches Patentamt

European Patent Office

Office européen des brevets

(11) Publication number: **0 324 834 B1**

# EUROPEAN PATENT SPECIFICATION

(45) Date of publication of patent specification: **08.11.95**  (51) Int. Cl.⁶: **C12Q 1/70**, G01N 33/53, B65D 69/00

(21) Application number: **88906559.5**

(22) Date of filing: **28.06.88**

(86) International application number:
**PCT/US88/02178**

(87) International publication number:
**WO 89/00207 (12.01.89 89/02)**

The file contains technical information submitted
after the application was filed and not included in
this specification

(54) **METHOD FOR RAPID AND SENSITIVE DETECTION OF HIV-I ANTIBODIES.**

(30) Priority: **13.07.87 DK 3622/87**
**21.09.87 US 99311**

(43) Date of publication of application:
**26.07.89 Bulletin 89/30**

(45) Publication of the grant of the patent:
**08.11.95 Bulletin 95/45**

(84) Designated Contracting States:
**AT BE CH DE FR GB IT LI LU NL SE**

(56) References cited:
**WO-A-88/08039**
**FR-A- 2 601 457**
**US-A- 4 452 901**
**US-A- 4 629 783**

**JOURNAL OF INFECTIOUS DISEASES, vol.
155, no. 1, January 1987; K. MARTIN et al., pp.
54-63&NUM;**

(73) Proprietor: **VERIGEN, INC.**
**92 South Street**
**Hopkinton, MA 01748 (US)**

(72) Inventor: **OSTHER, Kurt, B.**
**3341 Purdue Street**
**Dallas, TX 75225 (US)**
Inventor: **DYLL, Louis, M.**
**Route 4, Box 560**
**Rockwall, TX 75087 (US)**

(74) Representative: **Grünecker, Kinkeldey, Stock-
mair & Schwanhäusser Anwaltssozietät
Maximilianstrasse 58
D-80538 München (DE)**

EP 0 324 834 B1

JOURNAL OF CLINICAL IMMUNOLOGY, vol. 6, no. 3, May 1986; P.E. PHILLIPS et al., pp. 234-241&NUM;

METHODS OF ENZYMOLOGY, vol. 92, chapter 29, Academic Press, 1983; TSANG et al., pp. 377-391&NUM;

GENE ANALYSIS TECHNIOUES, vol. 1, no. 1, January 1984, New York, NY (US); D.A. JOHNSON et al., pp. 3-8&NUM;

## Description

TECHNICAL FIELD

The present invention relates to a rapid and sensitive assay method for the detection of antibodies to Human T-Cell Leukemia Virus-III (HIV-I) antigen, and diagnostic test kits for carrying out the assay method.

BACKGROUND OF THE INVENTION

Acquired immune deficiency syndrome (AIDS) was first recognized in approximately 1981, but the causative agent had not yet been identified. Intense research efforts resulted in the detection and isolation of HIV-I (previously known as HTLV-III/LAV), the retrovirus identified as the etiologic agent of AIDS.

The virus is currently believed to be transmitted through intimate sexual contact and blood. Thus far, epidemiologic evidence indicates that food, water, insects and casual contact are not disease vectors.

The AIDS virus acts by crippling the body's immune system. Particularly, HIV-I selectively attacks T4 lymphocytes, one of the subpopulations of lymphocytes that constitute the immune system. Infection with HIV-I results in both a reduction in the number and a change in the function of the targeted T4 lymphocytes with the eventual collapse of the immune system.

The groups at highest risk of infection with HIV-I include homosexual and bisexual men and abusers of injected drugs. Other predictable high risk groups are women artificially inseminated with sperm from infected donors and sexual partners of those in the AIDS risk groups. Recipients of blood transfusions and blood products are also at risk of contracting AIDS.

Organ transplant recipients are also a high risk group. Recognizing organ transplantation as a potential avenue for the spread of AIDS, testing for HIV-I antibodies is now performed on all organ donors. With the exception of some kidney transplants it is often difficult to predict in advance the source of a donated organ. For example, victims of fatal automobile accidents are potential organ donors, and as the time and place of accidents are not foreseeable, pre-transplant testing presents a problem. Screening is thus difficult in this and similar situations and the need for 24 hour testing facilities and, more importantly, on sits testing is apparent. Further, in view of the limited useful life of a donated organ once removed from the host, the need for a rapid assay method is evident.

Present methods far the detection of antibodies to the AIDS virus include the so-called enzyme-liked immunosorbance assay (ELISA), the Western Blot assay and the immunofluorescent assay. Such assays are described in Sarngadharan, M.G. et al., Science 224:506 (1983) (ELISA); Tsang et al., In: Methods of Enzymology, Vol. 92, Chapter 29, 1983, Academic Press and Safai, B. et al., Lancet 1:1438 (1984) (Western Blot), and Essex, M. et al., Science 220:859 (1983) (immunofluorescent techniques). The established method for blood donor screening is to first carry out an ELISA, followed by confirmation of positives by Western Blot.

The ELISA technique involves reacting a test simple with an antigen reagent generally obtained from disrupted whole or density-banded HIV-I. Typically, the HIV-I is coated onto wells of a microtiter plate. After washing to remove unbound antibodies, goat-antihuman IgG antiserum conjugated with horseradish peroxidase is added to the wells and incubated. After an appropriate incubation period, an enzyme substrate is added to the mixture and a detectable, measurable color product is formed in the presence of antibodies to HIV-I.

In the Western Blot assay, on the other hand, the HIV-I antigen is electrophoretically resolved on SDS-polyacrylamide gels, each 8x10 cm gel being loaded with 50-100 $\mu$g of protein. The resulting protein bands are electro-transferred to nitrocellulose paper. Detection of antibodies to HIV-I is then carried out by either solid phase strip radioimmunoassay techniques or by ELISA. Each of these methods includes an overnight incubation, and thus, an overall test time of about 20 hours.

The established screening procedure is not entirely satisfactory because of the time required to obtain results. This is particularly true in the organ transplant situation, especially for the heart and liver. These two organs have maximum cold ischemic times of 4 and 8 hours, respectively. Generally, ELISA takes about 4 hours and the Western Blot which, as noted, includes an overnight incubation period, requires about 20 hours. As can be appreciated, with conventional techniques test results would not be obtained within the maximum ischemic times for the heart or liver.

In "Journal of Infectious Diseases", vol. 155, page 54 to 63, 1987, a method for the detection of antibodies to HIV-1 retrovirus using the normal Western Blot procedure is disclosed, said method involving an intermediate step of blocking the nitrocellulose filter loaded with HIV-antigen by incubation overnight in 5% (w/v) non-fat dry milk, 0.01% anti foam A and 0.002% thimerosal. A decrease in unspecific protein binding and an improvement of the results are demonstrated.

The so-called "Quick Western Blot" assay is a modification of the standard Western Blot assay and is the subject of WO application No. 87/07647. The test involves a Western Blot assay wherein the concentration of the sample being tested as well as the HIV-I antigen used is increased by at least 50% over the standard Western Blot. The technique then involves using the ELISA test to detect the bound antibodies. Because of the increased concentration of the antigen and test sample the resultant assay can be accomplished in as little as one hour and twenty minutes without the expected nonspecific protein noise. Furthermore the test can be done in areas not equipped to perform the other more time consuming tests.

Although the Quick Western Blot method is both quick and sensitive, it is an object of the present invention to provide an even more rapid and sensitive assay for the detection of antibodies to HIV-I.

SUMMARY OF THE INVENTION

The present invention involves a rapid and sensitive method for the detection of antibodies to HIV-I antigen which uses a modified Quick Western Blot technique. Specifically, the method comprises.

(a) contacting nitrocallulose paper containing blotted resolved HIV-1 antigen protein obtained from gel eleotrophoretically resolved viral lysate with a test sample diluted with a buffer and incubating the nitrocellulose paper and test sample to permit binding of antibodies present in the sample to the protein on the nitrocellulose paper wherein

(1) the viral lysate is electrotransferred to the nitrocallulose paper in a concentration at least 20%, but less than 40%, greater than the 50-100 μg of HIV-1 antigen protein per 10x16 cm electrophorasis gel utilized in the conventional Western Blot assay;

(2) the resolved HIV-I antigen protein is incubated in step (a)(1) in the presence or non-fat milk protein; and

(3) the test sample is diluted in buffer: to a concentration about 3 to 5 times greater than the concentration utilized in the Western Blot assay; or to a concentration 3 to 7 times greater than the 1:100 dilution utilized in the Western Blot assay in case of a serum sample;

(b) contacting the incubated nitrocellulose paper of step (a) with an enzyme conjugated antiserum reactive with said antibodies, and incubating to permit binding of the antiserum to said antibodies;

(c) contacting the incubated nitrocellulose paper of step (b) with an enzyme substrate specific for the enzyme of step (b), and incubating to thereby produce color;

(d) stopping the color producing reaction of step (c); and

(e) evaluating the amount of color produced as an indication of the presence of antibodies to the HIV-I viral lysate;

to complete the assay within 60 minutes.

Surprisingly, the milk proteins act to both enhance and accelerate specific protein binding; and to decrease unspecific protein binding. Consequently, test results are obtained in 50-60 minutes.

In the Western Blot assay the serum sample is diluted in order that non-specific binding of other proteins present therein will not produce overlapping protein banding interfering with the detection of antibodies to the HIV-I antigen. On the other band, employing the Quick Western Blot assay described in the aforesaid Osther application, protein concentrations at least 50% greater than those used in the conventional Western Blot have been successfully employed to detect antibodies to the AIDS virus while decreasing the test time by a factor of ten. It has now been found in accordance with the present invention that, when the HIV-I antigen is incubated in the presence of milk proteins, specific binding of the HIV-I antibody to the antigen is both enhanced and accelerated.

Utilizing the method of the present invention the concentration of the test sample may be increased relative to that used in the Western Blot Assay without impairing the selectivity of the assay, while the concentration of the sample may be decreased relative to that employed in the Quick Western Blot without either impairing the sensitivity or increasing the incubation times required for the assay. Indeed, the assay of the present invention results in better resolution than the Western Blot, enhanced specific binding relative to the Quick Western Blot, and markedly decreased incubation times than required to complete either the conventional Western Blot or Quick Western Blot assays. Furthermore, the assay of this invention produces significantly more distinct protein bands. This is extremely important for the reading of the test strips.

In accordance with the assay procedure, the milk proteins are present when the antibodies of HIV-I, possibly present in the sample to be tested, are reacted with the HIV-I antigen. It is believed that one or more of the milk proteins changes the electronic structure of the binding site on the antigen thereby increasing its affinity and specificity for the HIV-I antibody.

4

It will be understood that the preceding explanation is a hypothesis based on the limited information currently available, and is subject to revision based on later developments. Accordingly, the present invention is not restricted thereto.

The method of the invention applies blotting techniques but uses at least 20%, preferably about 20% to 40%, more HIV-I viral lysate than conventional Western Blot. In particular, from 60 to about 120 $\mu$g of HIV-I antigen protein/10x16cm gel is used in the method of the invention, as compared with the 50-100$\mu$g of protein/10x16cm gel used in conventional Western Blot. Concentrations of test sample are also increased at least 3 times, preferably about 5 to 7 times, than that used in conventional western Blot.

The method of the invention, like the conventional Western Blot, employs an electrophoretically resolved antigen subsequently electrotransferred to test sheets or strips. The increase in the antigen concentration of the lysate together with the increased sample concentration and the introduction of milk proteins, facilitate a drastic reduction in incubation times. In accordance with the present method, test results are obtained in 50-60 minutes, that is 1/30 the time required to perform the Western Blot assay and 3/4 the time required for the Quick Western Blot assay. The method hereof is, therefore, referred to as the "Quick Western Blot II" technique.

The method involves electrophoretically resolving HIV-I antigen lysate and subsequently electrotransferring the resolved antigen onto nitrocellulose paper. This paper is incubated with the sample to be tested in order to detect any HIV-I antibodies present. The incubation step is performed in the presence of the milk proteins.

The nitrocellulose bound antigen/antibody complex is incubated with enzyme conjugated antiserum and developed with an enzyme substrate (color change indicator). The resultant colored bands are then visually compared with positive and negative controls in order to ascertain the presence of HIV-I antibodies.

In a preferred embodiment of the present invention, a diagnostic test kit is provided which permits on site testing for antibodies to AIDS virus. The kit comprises a set of control tubes for positive and negative references as well as a dilution tube with buffer for dilution of the test sample. The contents of these tubes are added to reaction tubes containing nitrocellulose test strips containing resolved HIV-I antigen and incubated. The resultant strips are incubated with the supplied enzyme conjugated antiserum used to detect whether any bound antibodies are present. The strips are then ultimately developed for color with a color change indicator.

The diagnostic kit of the invention also includes predeveloped positive and negative reference strips and reagent control strips for evaluating the test results by visual comparison with the test strips. The visible protein bands on the test sample strip with the positive reference strip should test positive for HIV-I antibodies (as shown from the prespecified protein bands). The negative reference strip should test negative for the HIV-I antibody. Accordingly, reading the results of the test is facilitated and the need for specially trained personnel is virtually eliminated. On site testing is particularly important in the organ transplant situation because screening for AIDS can now be performed on a 24 hour basis at virtually any location, without specially trained personnel, and test results can be obtained in 50-60 minutes, comfortably within the life span of ischemic organs.

## DETAILED DESCRIPTION OF THE INVENTION

### Resolution Of The HIV-I Antigen

In accordance with the method of the invention, HIV-I antigen concentrate is electrophoretically resolved. The HIV-I antigen concentrate may be obtained commercially, for example, as from Litton Bionetics as HIV-I viral lysate, Catalog no. 8464-15. The antigen concentrate is diluted in buffer to a protein concentration at least 20% but less than 40% greater than that utilized in conventional Western Blot. Preferably, the antigen concentrate is diluted in buffer to a protein concentration of about 60 to 120 ug per 10x16cm gel. The preferred buffer is 0.05M TRIS-HCl/50% glycerol, pH8, 2.5% SDS (sodium dodecyl sulfate) and 5% mercaptoethanol. Other buffers known to those skilled in the art are also suitable, such as 9M urea in 0.01M TRIS-HCl.

As noted above, the protein concentration of the antigen lysate used with the method of the invention is approximately 20 to as much as 40% higher than the 50-100ug/10x16cm gel typically used for conventional Western Blot. See pages 380 to 381 of the guidelines published by Tsang V.C., J. Peralta, R. Simons, In: Methods of Enzymology, Vol. 92, Chapter 29, 1983, Academic Press Inc., which sets forth the 50-100 $\mu$g 8x10cm gel workable range of protein concentrations used in the conventional Western Blot assay.

The antigen is generally first denatured by boiling, typically for about 5 minutes. Then, the denatured antigen is subjected to conventional gel electrophoresis of the type reported by Tsang et al, Methods in Enzymology, Vol. 92 (1983).

A tracking dye is preferably added to the diluted antigen to produce visible protein banding. The preferred dye is bromophenol blue. The dye is preferably prepared by dissolving 50 mg bromophenol blue in 8 ml of glycerol, plus 1 ml each of 0.5M TRIS-HCl at pH 8.0 and $H_2O$. Other dyes, known to those skilled in the art, may also be used.

Suitable gels for the electrophoresis are also prepared in accordance with the method of Tsang et al., Methods in Enzymology Vol. 92 (1983). A 10% resolving polyacrylamide gel with a 3% stacking gel (SDS-PAGE) is preferred because it resolves a molecular weight range of 12,000-160,000, thus embracing the proteins within the HIV-I viral lysate. However, a gradient SDS-PAGE gel can also be used. For example, a gradient ranging from 3.3%-20% polyacrylamide gel resolves the antigen in the desired molecular weight range. Preferably, a molecular weight marker is electrophoresed together with the HIV-I antigen. These marker materials serve to calibrate the gels and facilitate identification of the protein bands of specific molecular weights. Suitable molecular weight markers are commercially available, such as a Cytochrome C molecular weight system from United States Biochemical Corporation.

Electro-Transfer Of The Resolved Antigen

Subsequent to electrophoresis, the protein bands of the resolved antigen are electro-transferred preferably to nitrocellulose sheets, (e.g., those commercially available from Schleicher and Schuell, Inc. as Item no. BA 83 which is a roll of nitrocellulose paper having a 0.2 $\mu$m pore size). Other types of papers, known to those skilled in the art, such as diazo-type paper are also suitable. The electro-transfer of the protein bands is accomplished by means of the technique reported by Tsang et al., Methods in Enzymology Vol. 92, particularly page 378, and W. Van Raamsdonk, et al. J. Immunol. Methods 17:337 (1977).

In accordance with the present invention, the resolved HIV-I protein is incubated in the presence of milk proteins, preferably defatted proteins. (The presence of fat interferes with the test.) Suitable defatted milk proteins included Carnation® lowfat milk powder, but any defatted milk proteins as may be known are also useful. Such milk proteins are known to constitute about 60 to 90% casein, a phosphoprotein rich in serine; and from 10 to 40% of various other proteins including lactoalbumin, lactoglobulin, membrane globulin and a small amount of alkaline phosphatase, peroxidase catalase and xanthine dehydrogenase. The milk proteins may be introduced into the assay system in one of several ways.

According to a first embodiment of the invention, the nitrocellulose paper blotted with the resolved HIV-I antigen protein is coated with the milk proteins from a solution of buffer (e.g., PBS-Tween® 20), containing about 5 to 10% milk proteins for about 60 minutes. The nitrocellulose paper is subsequently dried at room temperature for a period of 30-60 minutes. After evaporation, the paper is washed in the buffer solution not containing milk proteins (e.g., PBS-Tween® 20) at room temperature For 1-5 minutes. The treated paper is then stored under humid conditions until use.

According to a second embodiment of the invention, the milk proteins are thoroughly mixed with and dissolved in the buffer solutions containing the test sample and controls. The controls and serum samples are then incubated with the nitrocellulose strips For 15 to 20 minutes. The liquid of each tube is discarded and the strips are washed with a PBS-Tween® buffer at pH 7.3-7.4. The washing cycle consists of four 1 minute washings.

According to a third embodiment of the invention, the milk proteins are precoated on the nitrocellulose strips and additionally mixed with and dissolved in the buffer solution.

The nitrocellulose sheets are then cut into strips approximately 2-2.5mm in width. Each strip, after appropriate labelling, is placed in a separate test tube or in aluminum foil for determination of antibodies to HIV-I viral lysate by the enzyme linked immunoassay of the invention. The nitrocellulose strips can also be placed in incubation trays for in house testing. It should be understood, however, that an uncut sheet can be placed in an incubation tray equipped with a pressing cover rather than cut into individual strips. This technique is also well-suited to in-house as opposed to on site testing. As can be appreciated, however, on site testing is facilitated by use of individual tubes. Also, placing the strips in individual tubes minimizes the need for handling during the assay procedure and thus, possible smearing of the fragile protein patterns with fingerprints. Using strips is also more economical than uncut sheets because less reagent is necessary to carry out the test.

Test samples, positive and negative references and reagent controls are added to the tubes containing the nitrocellulose strips blotted with resolved antigen. Test samples include, but are not limited to serum, semen and other body fluids. The positive reference is typically a sample known to contain antibodies to the HIV-I viral lysate. Positive references have been obtained from the Centers for Disease Control (CDC), Atlanta, Georgia. Alternatively, a positive reference may be made from any sample which has been standardized with a positive reference obtained from the CDC. Standardization typically means that the same test results were obtained in about 20 runs. The positive reference is diluted 1/200 (1 part positive reference to 200 parts buffer) in PBS (phosphate buffered solution)-Tween®, pH 7.2-7.4. Typically, 15μl of the positive reference is mixed with 3 ml PBS-Tween®.

The negative control is a sample known to be devoid of antibodies to HIV-I viral lysate, and is prepared by diluting 1/20 with PBS-Tween®, pH 7.2-7.4. Typically 150μl of a negative reference is mixed with 3 ml PBS-Tween®. Negative references have also been obtained from the CDC.

As the assay method of the invention is a qualitative rather than quantitative determination, the positive and negative references are used to evaluate the test results by comparison with the results obtained for test samples.

The reagent control is included as a quality control feature of the present invention and is used to assure accurate functioning of the test. Normally, the reagent control is the buffer used to dilute test samples and controls. Preferably, PBS-Tween®, pH 7.2-7.4 is used as the reagent control. However, the reagent control is not necessary during routine readings of the strips.

As indicated hereinabove, employing the milk protein treatment of the present invention, test samples may be used which are more concentrated than those used in conventional Western Blot, to accelerate the binding of antibodies to HIV-I viral lysate to the antigen contained in the strips. Typically, test samples are about three to five times more concentrated than samples tested by the Western Blot assay. On the other hand, test samples used in accordance with the present invention, need to be about 2.5 times less concentrated than the samples employed in the Quick Western Blot assay. The present method thus provides increased sensitivity, without markedly increased non-specific protein binding such as may occur employing very large test sample concentration.

For serum samples, three to seven times the concentration utilized in the conventional Western Blot assay is required. i.e., a dilution of one part serum to twenty parts buffer as compared to the 1:100 dilution factor used in the Western Blot assay. (See Tsang et al., Method of Enzymology, Vol. 92, 1983.) Theoretically, the actual dilution factor for particular samples may be varied, however, depending upon whether a specimen gives an extremely weak positive response. Studies indicate that five times the serum concentrations normally used with conventional Western Blot are unsuitable because the high amount of serum proteins, other than the antibodies being evaluated, interfere with the test. A PBS-Tween®, pH 7.2-7.4 buffer is preferred for the dilution of samples. Typically, 60μl of a serum sample is mixed with 3 ml of PBS-Tween®. But other known buffers may be substituted.

The strips are then incubated with the positive and negative references, controls and test samples at room temperature, preferably for about 10 to 20 minutes, to permit the binding of any antibodies to HIV-I present in the sample to the antigen in the nitrocellulose strips. A 20 minute incubation period is particularly preferred to insure optimum binding of weak positives.

Incubation With The Enzyme-Conjugated Antiserum

The liquid content of each tube is discarded, with the strips remaining in place in the tubes. The strips are then washed, preferably with PBS-Tween® buffer at pH 7.3. In particular, the washing cycle includes four 1 minute washings with PBS-Tween®. The strips are then incubated with an enzyne-conjugated anti-human IgG antiserum preferably for about 10 to 20 minutes, at room temperature, to permit binding of the enzyme conjugated antiserum to any antibody which bound to the antigen during the first incubation period. Preferably, goat anti-human IgG antiserum-horseradish peroxidase conjugate is employed, although other enzyme conjugated antisera as are known to those skilled in the art may be used. Again, a 20 minute incubation period is preferred.

Once more, the liquid content of each tube is discarded. The strips are then washed. Preferably the washing cycle includes four 1 minute washings with PBS-Tween® followed by one 1 minute washing with PBS.

Incubation With An Enzyme Substrate

Then, the strips are incubated with an enzyme substrate (color change indicator) for about 10 minutes at room temperature, for production of a color. The appropriate substrate for use with horseradish peroxidase enzyme is 3,3' diaminobenzidinetetrahydrochloride dihydrate (DAB). It should be understood, however, that substrate selection is dictated by the enzyme used. After the incubation period, the color reaction is stopped by addition of distilled $H_2O$ and the results determined, according to standard techniques, as reported by Tsang, et al., Methods in Enzymology, Vol. 92 (1983).

In accordance with the present invention, determination of the presence of antibodies to HIV-I can be accomplished in about 50-60 minutes because of the greatly reduced incubation times, totalling about 40 minutes, as compared with the Western Blot assay which requires at least 20 hours and the Quick Western Blot assay which takes 80 minutes.

The Diagnostic Test Kit

In accordance with a preferred embodiment of the invention, a self-contained diagnostic test kit is provided which permits "on site" screening for antibodies to HIV-I virus. The test kit includes a set of tubes containing positive and negative references and at least 1 buffer tube containing a predetermined volume of buffer to which the test sample is added in a predetermined amount to obtain a sample concentration from 3 to 7 times greater than that utilized in the Western Blot assay. The reference and control tubes are prediluted, and thus, the user need only dilute the test sample. A set of strip tubes is also provided, each tube containing a nitrocellulose strip containing resolved HIV-I antigen protein, electro-transferred from an SDS-PAGE gel loaded with from 20% to 40% higher antigen protein concentration than that used in conventional Western Blot. Preferably, the strips contain resolved HIV-I viral lysate, electrotransferred from an 10x16cm SDS-PAGE gel loaded with from 61-96$\mu$g of antigen protein compared to a range of 51-80$\mu$g for Western Blot, depending upon the relative amount of specific HIV proteins.

As indicated hereinabove, the milk protein additive is either pre-dissolved in the buffer solutions containing the test sample and the positive and negative references, or coated on the nitrocellulose strips containing the resolved HIV-I antigen protein. Alternatively, the milk proteins may be separately provided in powder form to be added to the buffer by the user; the resulting buffer solution can then be directly mixed with the test and reference samples, or used to coat the test strips.

In a preferred embodiment, the reference, control and sample tubes are numbered. The strip tubes are assigned numbers corresponding to those on the reference control and sample tubes. The strips are assigned numbers corresponding to the tubes in which they are placed. This type of numbering system avoids inadvertent mix-ups which can destroy the accuracy of the assay. As can be appreciated, if the top of a tube containing a positive sample is placed on a tube containing a negative sample, it is likely to obtain a false positive result.

The kit also contains vials of enzyme-conjugated antiserum reagent, substrate or color change indicator, two washing buffers and solution for terminating the color reaction. Preferably, goat anti-human IgG antiserum-horseradish peroxidase is used as the enzyme conjugated anti-serum reagent. The preferred substrate, reaction terminating agent and washing buffers are DAB, distilled H$_2$O, and PBS-Tween® and PBS, respectively.

Preferably, pre-developed positive and negative reference strips and reagent control strips are provided in the kit. These controls are prepared in substantially the same manner as previously described except that after developing, the strips are air dried. The predeveloped strips are used to evaluate the test results by a visual comparison with the test strips after completion of a color reaction. The reagent control as noted, is provided to assure the accurate functioning of the reagents. The predeveloped reference and control strips are a significant feature of the present invention because they facilitate reading the assay results and practically eliminate the need for a skilled technician to evaluate the results.

Also, as the kit is self-contained, no laboratory equipment is needed. The advantages of such a kit are apparent, as it facilitates screening for HIV-I antibodies at any time and virtually at any place, including remote geographic areas and those locations lacking a 24 hour testing facility. As aforementioned, this is of utmost importance in certain organ transplantation situations.

It has been reported that with HIV-I infection the major immune reactivity is directed togp41, a 41,000 molecular weight protein and especially gp120 and gp160, 120,000 and 160,000 molecular weight proteins, respectively, which are proteins believed to be envelope proteins of the virus. Thus, gp41, gp120 and/or gp160 bands are of critical significance in the present method. Also of importance is p24, a 24,000 molecular weight protein. Typically, the test is considered positive if activity is recorded at the p24 and gp41 bands and/or the gp120 and/or gp160 bands. Accordingly, proper resolution of the HIV-I antigen lysate is vital. The amount of protein subjected to electrophoresis is related to the distinctiveness of the resulting bands. Obviously, it is desirable to obtain distinct banding at the critical points, particularly, gp20, gp160 and gp41 as well as p24 with few or no noise bands. The method of present invention results in better resolution of the important gp160 and gp120 bands than either of the conventional and Quick Western Blot techniques. The presence of those higher molecular weight proteins provide a more accurate indication of the presence of the AIDS retrovirus in the tested sample since these envelope proteins are considered to be the most reliable bands in the evaluation of the test strips.

The protein bands are much clearer and better resolved in the present invention as compared to the other Western Blots, resulting in greater ease in interpreting the results.

One assay (Quick Western Blot I) was conducted in accordance with the method set forth in copending WO Application No. 87/07647. The SDS-PAGE gel/10x16cm was loaded with 76.5 $\mu$g HIV lysate and the total reaction time was 70 minutes.

Another assay (Quick Western Blot II) was conducted in accordance with the method described herein. The SDS-PAGE gel was loaded with appr. 67 $\mu$g of HIV lysate. The milk protein was precoated on the nitrocellulose strips and added to the buffer containing the samples. The total reaction time was 40 minutes. (Note chat the viral lysate used in each of the assays were obtained from different batches).

The following specific examples of the Quick western Blot II Assay and its use of milk proteins are further illustrative of the nature of the present invention, although it is understood that the invention is not limited thereto.

Example 1

The Quick Western Blot II Assay, Dissolving the Milk Proteins in the Buffer Solutions.

HIV-I antigen concentrate was electrophoretically resolved in the molecular weight range of 12,000-160,000.

The HIV lysate antigen was electro-transferred to nitrocellulose paper. The paper was then cut into strips and placed in separate test tubes.

0.15g of Carnation® milk protein powder was dissolved in each 3ml buffer solution containing the sample and controls, by turning end over end for 5-10 times to allow complete mixing with the milk proteins. The controls and serum samples were then incubated with the nitrocellulose strips for 15 minutes. The liquid of each tube was discarded and the straps were washed with PBS-Tween buffer at pH 7.3-7.5. The washing cycle consisted of four 1 minute washings.

The strips were then incubated with an enzyme conjugated anti human IgG antiserum (1:500) for 15 minutes at room temperature. The liquid was again discarded and the strips were washed four times with PBS-Tween® followed by a one minute washing with PBS alone.

The strips were incubated for 10 minutes with horseradish peroxidase and its substrate 3,3' diaminobenzidinetetrahydrochloride dihydrate (DAB). The color reaction was stopped by the addition of distilled $H_2O$ and the results were evaluated by comparing the test strips with the pre-developed positive, negative and reagent strips provided with the kit, and the assay was completed within 50 minutes of the initial treatment of the test sample.

Example II

The Quick Western Blot II Assay, Coating the Antigen Bound Nitrocellulose Strips with Milk Protein.

HIV-I antigen concentrate corresponding to 61 $\mu$g/10x16 cm gel was electrophoretically resolved in the molecular weight range of 12,000-160,000.

The HIV lysate antigen was electro-transferred to nitrocellulose paper. The paper was then coated in a bath equipped with magnetic stirring with a PBS-Tween® 20 buffer solution containing nonfat milk proteins.

The PBS-Tween buffer contained 5% Carnation® milk proteins. This was achieved by mixing 5g of the powder with 100ml of the buffer.

The nitrocellulose paper was subsequently dried at room temperature for a period of 5-30 minutes. After evaporation, the paper was washed in PBS-Tween®-20 at room temperature for 2 minutes. The strips were then stored under humid conditions until use.

The procedure of Example I was then followed beginning with the initial incubation of the nitrocellulose strips.

A study was performed comparing the QWB II method described herein with the conventional Western Blot and ELISA techniques. The results of the tests run on 338 serum samples is reported in Table I. As shown in Table I, the QWB II assay method of the present invention is as reliable as the conventional Western Blot assay. In fact, at least one sample which was inconclusive using the Western Blot was shown to be positive using the Quick western Blot II Assay. The advantages of the Quick Western Blot II are apparent.

The presence of antibodies to HIV-I can be detected in 50 minutes. The rapidity with which the test can be performed is enormously important to organ transplant recipients, since donated organs have a limited usable life span outside the body, and in trauma cases where immediate surgery is needed. The Quick Western Blot II, through the use of milk proteins, results in both enhanced and accelerated specific protein binding. Furthermore, less unspecific binding of proteins occur. The certainty with which the specific bands can be detected facilitates the diagnosis of AIDS and probably also the provides useful information as to the stage of the HIV infection antibodies to different molecular weight antigen proteins may be indicative of various stages in the progression of the disease. Table II provides information about correlating the presence of specific protein bands with various stages in the progression of the disease.

Additionally, and apart from the organ donor situation, utilizing the method and diagnostic tent kit of the invention, hospitals can obtain fast and accurate results on patients suspected of HIV-I infection, and thus expedite treatment. Moreover, if AIDS is diagnosed, hospital personnel can more readily adopt necessary precautions and minimize accidental viral contamination.

While preferred embodiments of the invention have been described, it will be apparent to those of ordinary skill in the art that various changes and modifications may be made therein without departing from the spirit and scope of the invention. Accordingly, the above description should be construed as illustrative, and not in a limiting sense, the scope of the invention being defined by the following claims.

TABLE I

| Study Comparing Conventional Western Blot and ELISA with Quick Western Blot II | | | |
|---|---|---|---|
| | HIV Ab ELISA | HIV Ab WB | HIV Ab QWB2 |
| POSITIVE FOR ANTIBODY | 208 | 120 | 130 |
| INCONCLUSIVE RESULTS | - | 12 | 11 |
| NEGATIVE RESULTS | 130 | 197 | 197 |

338 serum specimens obtained from Baylor University Medical center and from various Laboratories in the Dallas Ft. Worth Metroplex Area, were tested for the presence of antibodies to HIV-I with ELISA screening with Behring Processor 2 for HIV antibody using Electronucleonic HIV Ab Test kits, with Quick Western Blot 2 (QWB2 or RapWB) and with confirmatory conventional Western Blot technique. The QWBII was performed in accordance with the method described herein. The milk proteins were precoated on the nitrocellulose strips and mixed into the buffer solutions containing the samples and controls.

## TABLE II

### HIV Antigen/Antibody Rapid Western Blot Patterns
### (Correlation with Stage and Prognosis)

| CLINICAL STAGE | TEST RESULTS |
| --- | --- |
| Acute Infection | HIV Antigenemia (First) |
| | IgM Antibodies to HIV (second) (ELISA Negative) |
| | IgG Anti p24/55 & gp160/120 (Third)  (In some cases ELISA Negative in the start) |
| | IgG Anti gp41 (Fourth) |
| Dormant but still | HIV Antigenemia variable |
| Infectiv. | IgM Antibodies often present within 1. month after infection (ELISA Negative) |
| (after 1 to 2 months following infection) | IgG anti p24/55 present |
| | IgG anti gp160/120 present |
| | IgG anti gp41 present |
| | IgG anti p64/53 present |
| | IgG anti Reverse Transcriptase present |

# EP 0 324 834 B1

| AIDS and pneumosystis | HIV Antigenemia present |
|---|---|
| | IgG anti p 24/55 low or absent |
| | IgG anti gp41 low or absent |
| | **IgG anti gp160 present at low levels** |
| | IgG anti p53 low or absent |
| | **IgG anti p64 present at low levels** |
| Kaposi's Sarcoma | HIV Antigenemia present in many cases. |
| | IgG anit p24/55 low or absent |
| | IgG anti gp41 low or absent |
| | **IgG anti gp120 present at low levels** |
| | **IgG anti gp160 present at low levels** |
| | **IgG anti p53 present at low levels** |
| | **IgG anti p64 present at low levels** |

Information for Table II was obtained from the following sources:

1. Pan, L-2, Cheng - Mayer, C., Levy, JA J. Infect. Dis. (155) 626-632 (1987).
2. Lelie, P.N., Van der Poel, C.L., Reesink, H.W. Lancet (I) p632 (1987).
3. Kumar, P., Pearson, J., Martin, D.H. et al. Ann Intern. Med. (106) 244-245 (1987).
4. Wall, R.A., Denning, D.W., Amos, A. Lancet (I) p566 (1987).
5. Weber, J.M., Clapham, P.R. Lancet (I) 119-122 (1987).

**Claims**

**1.** A method for the detection of antibodies to HIV-I retrovirus, comprising:

(a) contacting nitrocellulose paper containing blotted resolved HIV-I antigen protein obtained from gel electrophoretically resolved viral lysate with a test sample diluted with a buffer and incubating the nitrocellulose paper and test sample to permit binding of antibodies present in the sample to the protein on the nitrocellulose paper wherein

(1) the viral lysate is electrotransferred to the nitrocellulose paper in a concentration at least 20%, but less than 40%, greater than the 50-100 $\mu$g of HIV-1 antigen protein per 10x16 cm electrophoresis gel utilized in the conventional Western Blot assay;

(2) the resolved HIV-I antigen protein is incubated in step (a)(1) in the presence of non-fat milk protein; and

(3) the test sample is diluted in buffer to a concentration about 3 to 5 times greater than the concentration utilized in the conventional Western Blot assay; or to a concentration at least 3, but less than 7, times greater than the 1:100 dilution of the test sample utilized in the Western Blot assay in case of a serum sample;

(b) contacting the incubated nitrocellulose paper of step (a) with an enzyme conjugated antiserum reactive with said antibodies, and incubating to permit binding of the antiserum to said antibodies;

(c) contacting the incubated nitrocellulose paper of step (b) with an enzyme substrate specific for the enzyme of step (b), and incubating to thereby produce color;

(d) stopping the color producing reaction of step (c); and

(e) evaluating the amount of color produced as an indication of the presence of antibodies to the HIV-I viral lysate;

to complete the assay within 60 minutes.

2. The method of claim 1, wherein said non-fat milk protein comprises from 60 to 90% by weight casein, and from 10 to 40% by weight of a material selected from the group consisting of lactoglobulin, membrane globulin, alkaline phosphatase, peroxidase catalase, xanthine dehydrogenase, and mixtures thereof.

3. The method of claim 1, wherein the nitrocellulose paper containing the blotted resolved HIV-1 antigen is coated with non-fat milk protein prior to incubation with the test sample in step (a).

4. The method of claim 1, wherein the non-fat milk protein is mixed with the buffer used to dilute said test sample.

5. The method of claim 1, wherein said antigen lysate has a protein concentration of between 60-100 $\mu$g per 10x16 cm gel.

6. The method of claim 1, wherein said test sample is serum.

7. The method of claim 1, in which step (a) is repeated with at least one positive control (a sample containing HIV-1 antibodies) and et least one negative control (a sample that is devoid of HIV-1 antibodies) in place of the test sample, the reaction product formed thereby is subjected to steps (b-e), and the colors produced are compared as standards with the color produced from the test sample, to evaluate the presence of antibodies to the HIV-1 retrovirus in the test sample.

8. The method of claim 1, wherein the resolved HIV-I antigen protein of step (a) (1) is treated with non-fat milk protein by

(1) coating the blotted nitrocellulose paper produced in step (a) (1) with 3 to 10% non-fat milk protein, or

(2) incorporating from 3 to 10% non-fat milk protein in the buffer utilized in step (a) (2)

9. The method of claim 8, in which step (a) is repeated with at least one positive control (a sample containing HIV-1 antibodies) and one negative control (a sample that is devoid of HIV-1 antibodies) in place of the test sample, the reaction product formed thereby is subjected to steps (b-e), and the colors produced are compared as standards with the color produced from the test sample, to evaluate the presence of antibodies to the HIV-1 retrovirus in the test sample.

10. The method of claim 8, wherein the test sample is mixed with a buffer selected from the group consisting of PBS-Tween, TRIS buffers, TBS buffers, urea buffers, and polyethylene glycol in saline or distilled water.

11. The method of claim 1, wherein each incubation step is effected in no more than 20 minutes.

12. The method of claim 11, in which step (a) is repeated with at least one positive control (a sample containing HIV-1 antibodies) and one negative control (a sample that is devoid of HIV-1 antibodies) in place of the test sample, the reaction product formed thereby is subjected to steps (b-e), and the colors produced are compared as standards with the color produced from the test sample, to evaluate the presence of antibodies to the HIV-1 retrovirus in the test sample.

13. A diagnostic test kit for the detection of AIDS specific antibodies comprising:

EP 0 324 834 B1

(a) a set of control tubes comprising positive and negative references;

(b) at least one reagent control tube;

(c) at least one dilution tube containing a predetermined volume of buffer for dilution of test samples to obtain a concentration of test samples about 3 to 5 times greater than the concentration utilized in the Western Blot assay; or to a concentration 3 to 7 times greater than the 1:100 dilution of the test sample utilized in the Western Blot assay in the case of the serum sample;

(d) a set of tubes containing nitrocellulose test strips containing resolved HIV-1 antigen, said antigen being obtained from gel electrophoretically resolved viral lysate, wherein the resolved HIV-1 antigen is present in a concentration at least 20% to 40% greater than the 50-100 $\mu$g of antigen protein per 10x16 cm electrophoresis gel utilized in the conventional Western Blot assay;

(e) a non-fat milk protein reagent for binding with the resolved HIV-1 antigen, said reagent being dissolved in the buffer in dilution tube (c) or coated on the nitrocellulose test strips in tubes (d);

(f) an enzyme conjugated antiserum for reacting with the antibody-antigen complex;

(g) a color change indicator to ascertain whether the HIV-1 antibodies are present; and

(h) predeveloped positive and negative reference strips and reagent control strips for evaluating the results of the test by visually comparing the predeveloped strips with the test strips after completion of a color change reaction.

**14.** The diagnostic test kit of claim 13, wherein the electrophoretically resolved antigen has a concentration of 60 to 100 $\mu$g HIV protein/10x16 cm gel.

**15.** The diagnostic test kit of claim 13, wherein the dilution tube contains 3 to 10% nonfat milk proteins.

**16.** The diagnostic test kit of claim 15, wherein the nonfat milk proteins comprise from 60 to 90% by weight casein, and from 10 to 40% by weight of a material selected from the group consisting of lactoglobulin, membrane globulin, alkaline phosphatase, peroxidase catalase, xanthine dehydrogenase, and mixtures thereof.

**Patentansprüche**

**1.** Verfahren zum Nachweis von Antikörpern gegen das HIV-I-Retrovirus, umfassend:

(a) Inkontaktbringen von Nitrozellulosepapier, enthaltend darauf aufgebrachtes aufgetrenntes HIV-I-Antigenprotein, erhalten von einem gelelektrophoretisch aufgetrennten Viruslysat, mit einer Testanalysenprobe, die mit einem Puffer verdünnt ist, und Inkubieren des Nitrozellulosepapiers und der Testanalysenprobe, um die Bindung von Antikörpern, die in der Analysenprobe vorhanden sind, an das Protein auf dem Nitrozellulosepapier zu ermöglichen, worin

(1) das Viruslysat per Elektrotransfer auf das Nitrozellulosepapier übertragen wird in einer Konzentration von mindestens 20 %, aber weniger als 40 %, größer als die 50-100 $\mu$g von HIV-I-Antigenprotein pro 10 x 16 cm Elektrophoresegel, das in einem herkömmlichen Western-Blot-Assay verwendet wird;

(2) das aufgetrennte HIV-I-Antigenprotein wird in Schritt (a) (1) in der Anwesenheit von fettfreiem Milchprotein inkubiert; und

(3) die Testanalysenprobe wird in einem Puffer verdünnt auf eine Konzentration, die ungefähr 3-5 mal größer ist als die Konzentration, die in dem herkömmlichen Western-Blot-Assay verwendet wird; oder auf eine Konzentration, die mindestens 3 mal aber weniger als 7 mal größer ist als die 1:100 Verdünnung der Testanalysenprobe, die in dem Western-Blot-Assay im Falle einer Serumanalysenprobe verwendet wird;

(b) Inkontaktbringen des inkubierten Nitrozellulosepapiers aus Schritt (a) mit einem enzymkonjugierten Antiserum, das mit den Antikörpern reaktionsfähig ist, und Inkubieren, um die Bindung des Antiserums an die Antikörper zu ermöglichen;

(c) Inkontaktbringen des inkubierten Nitrozellulosepapiers aus Schritt (b) mit einem Enzymsubstrat, das spezifisch ist für das Enzym aus Schritt (b), und Inkubieren, um dadurch eine Farbe zu erzeugen;

(d) Abstoppen der Farberzeugungsreaktion von Schritt (c); und

(e) Ermitteln der Menge an hergestellter Farbe als einen Hinweis auf die Anwesenheit von Antikörpern gegen das HIV-I-Viruslysat;

um den Assay innerhalb von 60 Minuten zu vervollständigen.

14

2. Verfahren nach Anspruch 1, worin das fettfreie Milchprotein 60 bis 90 Gew.-% Casein und 10 bis 40 Gew.-% eines Materials umfaßt, das ausgewählt wird aus der Gruppe bestehend aus Lactoglobulin, Membranglobulin, alkalischer Phosphatase, Peroxidasekatalase, Xyanthindehydrogenase und Mischungen davon.

3. Verfahren nach Anspruch 1, worin das Nitrozellulosepapier, das das aufgebrachte aufgetrennte HIV-I-Antigen enthält, beschichtet wird mit fettfreiem Milchprotein vor der Inkubation mit der Testanalysenprobe in Schritt (a).

4. Verfahren nach Anspruch 1, worin das fettfreie Milchprotein mit dem Puffer, der zum Verdünnen der Testanalysenprobe verwendet wird, gemischt wird.

5. Verfahren nach Anspruch 1, worin das Antigenlysat eine Proteinkonzentration zwischen 60 bis 100 $\mu$g pro 10 x 16 cm Gel besitzt.

6. Verfahren nach Anspruch 1, worin die Testanalysenprobe ein Serum ist.

7. Verfahren nach Anspruch 1, bei dem Schritt (a) wiederholt wird mit mindestens einer Positivkontrolle (einer Analysenprobe enthaltend HIV-I-Antikörper) und mindestens einer Negativkontrolle (einer Analysenprobe, die frei ist von HIV-I-Antikörpern) anstelle der Testanalysenprobe, das dadurch gebildete Reaktionsprodukt wird Schritten (b) bis (e) unterzogen, und die erzeugten Farben werden als Standards mit der Farbe verglichen, die von der Testanalysenprobe erzeugt wird, um die Anwesenheit von Antikörpern gegen das HIV-I-Retrovirus in der Testanalysenprobe zu ermitteln.

8. Verfahren nach Anspruch 1, worin das aufgetrennte HIV-I-Antigenprotein aus Schritt (a) (1) behandelt wird mit fettfreiem Milchprotein durch
    (1) Beschichten des Nitrozellulosepapiers, hergestellt durch die Übertragung in Schritt (a) (1), mit 3 bis 10 % fettfreiem Milchprotein, oder
    (2) Einbringen von 3 bis 10 % fettfreiem Milchprotein in den in Schritt (a) (2) verwendeten Puffer.

9. Verfahren nach Anspruch 8, in dem Schritt (a) wiederholt wird mit mindestens einer Positivkontrolle (einer Analysenprobe, enthaltend HIV-I-Antikörper) und einer Negativkontrolle (einer Analysenprobe, die frei ist von HIV-I-Antikörpern) anstelle der Testanalysenprobe, das dadurch gebildete Reaktionsprodukt wird Schritten (b) bis (e) unterzogen, und die erzeugten Farben werden als Standards verglichen mit der von der Testanalysenprobe erzeugten Farbe, um die Anwesenheit von Antikörpern gegen das HIV-I-Retrovirus in der Testanalysenprobe zu ermitteln.

10. Verfahren nach Anspruch 8, worin die Testanalysenprobe gemischt wird mit einem Puffer ausgewählt aus der Gruppe bestehend aus PBS-Tween, TRIS-Puffern, TBS-Puffern, Harnstoffpuffern und Polyethylenglycol in Kochsalz oder destilliertem Wasser.

11. Verfahren nach Anspruch 1, worin jeder Inkubationsschritt nicht mehr als 20 Minuten durchgeführt wird.

12. Verfahren nach Anspruch 11, in dem Schritt (a) wiederholt wird mit mindestens einer Positivkontrolle (einer Analysenprobe, enthaltend HIV-I-Antikörper) und einer Negativkontrolle (einer Analysenprobe, die frei ist von HIV-I-Antikörpern) anstelle der Testanalysenprobe, das dadurch gebildete Reaktionsprodukt wird Schritten (b) bis (e) unterzogen, und die erzeugte Farbe wird als Standards verglichen mit der von der Testanalysenprobe erzeugten Farbe, um die Anwesenheit von Antikörpern gegen das HIV-I-Retrovirus in der Testanalysenprobe zu ermitteln.

13. Ein diagnostischer Testkit für den Nachweis von AIDS-spezifischen Antikörpern, umfassend:
    (a) einen Satz an Kontrollgefäßen, umfassend positive und negative Referenzen;
    (b) mindestens ein Kontrollreagenzgefäß;
    (c) mindestens ein Verdünnungsgefäß, enthaltend ein vorab bestimmtes Volumen an Puffer zum Verdünnen der Testanalysenprobe, um eine Konzentration der Testanalysenproben von ungefähr 3 bis 5 mal größer als die Konzentration, die in dem Western-Blot-Assay verwendet wird, zu erhalten; oder auf eine Konzentration von 3 bis 7 mal größer als die 1:100 Verdünnung der Testanalysenprobe, die in dem Western-Blot-Assay in dem Fall der Serumanalysenprobe verwendet wird;

(d) einen Satz an Gefäßen, enthaltend Nitrozelluloseteststreifen, enthaltend aufgetrenntes HIV-I-Antigen, wobei das Antigen von gelelektrophoretisch aufgetrenntem Viruslysat erhalten wurde, worin das aufgetrennte HIV-I-Antigen in einer Konzentration von mindestens 20 % bis 40 % größer ist als die 50 bis 100 µg von Antigenprotein pro 10 x 16 cm Elektrophoresegel, das in dem herkömmlichen Western-Blot-Assay verwendet wird;

(e) ein fettfreies Milchproteinreagenz zum Binden mit dem aufgetrennten HIV-I-Antigen, wobei das Reagenz gelöst ist in dem Puffer in Verdünnungsgefäß (c) oder beschichtet ist auf die Nitrozelluloseteststreifen in Gefäßen (d);

(f) ein enzymkonjugiertes Antiserum zum Reagieren mit dem Antikörper-Antigenkomplex;

(g) ein Farbwechselindikator zum Sicherstellen, ob die HIV-I-Antikörper vorhanden sind; und

(h) vorentwickelte positive und negative Referenzstreifen und Reagenzkontrollstreifen zum Abschätzen der Ergebnisse des Tests durch visuelles Vergleichen der vorentwickelten Streifen mit den Teststreifen nach dem Beenden einer Farbänderungsreaktion.

14. Diagnostischer Testkit nach Anspruch 13, worin das elektrophoretisch aufgetrennte Antigen eine Konzentration von 60 bis 100 µg HIV-Protein/10 x 16 cm Gel besitzt.

15. Diagnostischer Testkit nach Anspruch 13, worin das Verdünnungsgefäß 3 bis 10 % fettfreie Milchproteine enthält.

16. Diagnostischer Testkit nach Anspruch 15, worin die fettfreien Milchproteine 60 bis 90 Gew.-% Casein und 10 bis 40 Gew.-% eines Materials umfaßt, das ausgewählt wird aus der Gruppe, bestehend aus Lactoglobulin, Membranglobulin, alkalische Phosphatase, Peroxidasekatalase, Xanthindehydrogenase und Mischungen davon.

**Revendications**

1. Un procédé de détection d'anticorps contre le rétrovirus HIV-1, comprenant:

(a) La mise en contact d'un papier de nitrocellulose contenant une protéine d'antigène HIV-1 résolue buvardée obtenue à partir de lysats viraux auxquels on a fait subir une résolution électrophorétique avec un échantillon expérimental dilué avec un tampon, et l'incubation du papier de nitrocellulose et de l'échantillon expérimental pour permettre la liaison des anticorps présents dans l'échantillon à la protéine sur le papier de nitrocellulose dans lequel

(1) le lysat viral est électrotransféré sur le papier de nitrocellulose à une concentration au moins 20%, mais moins que 40%, spécifique aux 50-100 µg de protéine d'antigène HIV-1 par 10x16 cm de gel d'électrophorèse utilisés dans l'essai Western Blot classique;

(2) on fait incuber la protéine d'antigène HIV-1 résolue dans l'étape (a)(1) en présence d'une protéine de lait dégraissée ; et

(3) on dilue l'échantillon expérimental dans un tampon, à une concentration environ 3 à 5 fois supérieure à la concentration utilisée dans l'essai Western Blot classique; ou à une concentration au moins 3 fois, mais moins que 7 fois supérieure à la dilution 1:100 de l'échantillon expérimental utilisé dans l'essai Western Blot;

(b) la mise en contact du papier de nitrocellulose incubé de l'étape (a) avec un antisérum conjugué à l'enzyme réactif avec lesdits anticorps et l'incubation pour permettre la liaison de l'antisérum auxdits anticorps;

(c) la mise en contact du papier de nitrocellulose incubé de l'étape (b) avec un substrat enzymatique spécifique vis-à-vis de l'enzyme de l'étape (b) et l'incubation pour produire ainsi une couleur;

(d) l'arrêt de la réaction de production de couleur de l'étape (c); et

(e) l'évaluation de la quantité de couleur produite en tant qu'indication de la présence d'anticorps contre le lysat viral d'HIV-1;

pour achever l'essai en 60 minutes.

2. Procédé selon la revendication 1, dans lequel ladite protéine lactique non grasse comprend de 60 à 90% en poids de caséine, et de 10 à 40% en poids d'une matière choisie dans le groupe constitué par la lactoglobuline, la globuline membranaire, la phosphatase alcaline, la peroxydase catalase, la xanthine déshydrogénase et leurs mélanges.

**3.** Procédé selon la revendication 1, dans lequel on revit le papier de nitrocellulose contenant l'antigène d'HIV-1 résolu, buvardé, d'une protéine lactique non grasse avant incubation avec l'échantillon expérimental dans l'étape (a).

**4.** Procédé selon la revendication 1, dans lequel on mélange la protéine lactique non grasse avec le tampon utilisé pour diluer ledit échantillon expérimental.

**5.** Procédé selon la revendication 1 dans lequel ledit lysat d'antigène a une concentration protéique comprise entre 60 et 100 $\mu$g par gel de 10x16 cm.

**6.** Procédé selon la revendication 1, dans lequel ledit échantillon expérimental est du sérum.

**7.** Procédé selon la revendication 1, dans lequel ladite étape (a) est répétée avec au moins un témoin positif (échantillon contenant des anticorps anti-HIV-1) et au moins un témoin négatif (échantillon dépourvu d'anticorps anti-HIV-1) au lieu de l'échantillon expérimental, le produit de la réaction ainsi formé étant soumis aux étapes (b-e) et les couleurs produites étant comparées en tant que normes avec les couleurs produites à partir de l'échantillon expérimental, pour évaluer la présence d'anticorps contre le rétrovirus HIV-1 dans l'échantillon expérimental.

**8.** Procédé selon la revendication 1, dans lequel on traite la protéine d'antigène HIV-1 résolue de l'étape (a) (1) avec une protéine lactique non grasse en
(1) revêtant le papier de nitrocellulose buvardé produit dans l'étape (a)(1) avec 3 à 10% de protéine lactique non grasse; ou
(2) incorporant de 3 à 10% de protéine lactique non grasse dans le tampon utilisé dans l'étape (a)-(2).

**9.** Procédé selon la revendication 8, dans lequel l'étape (a) est répétée avec au moins un témoin positif (échantillon contenant les anticorps anti-HIV-1) et un témoin négatif (échantillon dépourvu d' anticorps anti-HIV-1) au lieu de l'échantillon expérimental, le produit de réaction ainsi formé est soumis aux étapes (b-e) et les couleurs produites sont comparées en tant que normes aux couleurs produites à partir de l'échantillon expérimental, pour évaluer la présence d'anticorps contre le rétrovirus HIV-1 dans l'échantillon expérimental.

**10.** Procédé selon la revendication 8, dans lequel on mélange l'échantillon expérimental avec un tampon choisit dans le groupe constitué par le STP-Tween, les tampons TRIS, les tampons TBS, les tampons d'urée et le polyéthylène glycol dans de l'eau distillée ou salée.

**11.** Procédé selon la revendication 1, dans lequel chacune des étapes de l'incubation est effectuée en pas plus de 20 minutes.

**12.** Procédé selon la revendication 11, dans lequel l'étape (a) est répétée avec au moins un témoin positif (un échantillon contenant des anticorps HIV-1) et un témoin négatif (échantillon qui est dépourvu d'anticorps anti-HIV-1) au lieu de l'échantillon expérimental, le produit de la réaction ainsi formé est soumis aux étapes (b-e), et les couleurs produites sont comparées en tant que normes avec la couleur produite à partir de l'échantillon expérimental, pour évaluer la présence d'anticorps contre le rétrovirus HIV-1 dans l'échantillon de test.

**13.** Nécessaire de test de diagnostic pour la détection d'anticorps spécifiques du SIDA comprenant:
(a) un ensemble de tubes témoins comprenant des références positives et négatives;
(b) au moins un tube réactif de témoin;
(c) au moins un tube de dilution contenant un volume prédéterminé de tampons pour la dilution d'échantillons expérimentaux pour obtenir une concentration d'échantillons expérimentaux d' environ 3 à 5 fois supérieure à la concentration utilisée dans l'essai Western Blot; ou à une concentration 3 à 7 fois supérieure à la dilution au 1:100 de l'échantillon expérimental utilisé dans l'essai Western Blot dans le cas de l'échantillon de sérum;
(d) un ensemble de tubes contenant des rubans de test de nitrocellulose contenant l'antigène HIV-1 résolu, ledit antigène étant obtenu à partir d'un lysat viral résolu par électrophorèse sur gel, dans lequel l'antigène HIV-1 résolu est présent à une concentration d'au moins 20% à 40% supérieure

aux 50-100 μg de protéine d'antigène par 10x16 cm de gel d'électrophorèse utilisés dans l'essai Western Blot classique;

(e) un réactif de protéine lactique non grasse pour la liaison avec l'antigène HIV-1 résolu, ledit réactif étant dissous dans le tampon, dans le tube à dilution (c) ou déposé sur les rubans de test de nitrocellulose dans les tubes (d);

(f) un antisérum conjugué à l'enzyme pour réaction avec le complexe anticorps-antigène;

(g) un indicateur de changement de couleur pour indiquer si les anticorps anti-HIV-1 sont présents; et

(h) des rubans de référence positifs et négatifs prédéveloppés et des rubans de réactif témoin pour évaluer les résultats des tests par comparaison à l'oeil nu des rubans prédéveloppés avec les rubans de test après la réalisation d'une réaction de changement de couleur.

14. Nécessaire de test de diagnostic selon la revendication 13, dans lequel l'antigène résolu électrophorétiquement a une concentration de 60 à 100 μg de protéine HIV/10x16 cm de gel.

15. Nécessaire de test de diagnostic selon la revendication 13, dans lequel le tube à dilution contient de 3 à 10% de protéines lactiques non grasses.

16. Nécessaire de test de diagnostic selon la revendication 15, dans lequel les protéines lactiques non grasses comprennent de 60 à 90% de caséine, et de 10 à 40% en poids d'une matière choisie dans le groupe constitué par la lactoglobuline, la globuline membranaire, la phosphatase alcaline, la peroxydase catalase, la xanthine déshydrogénase et leurs mélanges.